(19) **Europäisches Patentamt European Patent Office Office européen des brevets**

(11) **EP 2 356 993 B1**

(12) **FASCICULE DE BREVET EUROPEEN**

(45) Date de publication et mention
de la délivrance du brevet:
**21.06.2017 Bulletin 2017/25**

(51) Int Cl.:
***A61K 36/064*** *(2006.01)*    ***A61P 3/06*** *(2006.01)*

(21) Numéro de dépôt: **11152394.0**

(22) Date de dépôt: **27.01.2011**

(54) **Médicament pour la réduction de la cholestérolémie et de la triglycéridémie**

Medikament zur Herabsetzung des Cholesterol- und/oder Triglyceridspiegels.

Medicament for the reduction of cholesterolemia and/or triglyceridemia

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **27.01.2010 FR 1050537**

(43) Date de publication de la demande:
**17.08.2011 Bulletin 2011/33**

(73) Titulaire: **Biocodex
94250 Gentilly (FR)**

(72) Inventeurs:
• **Girard, Philippe
60280, MARGNY-LES-COMPIEGNE (FR)**
• **Verleye, Marc
60190, REMY (FR)**
• **Le Guern, Marie-Emmanuelle
60200, COMPIEGNE (FR)**
• **Hublot, Bernard
60200, COMPIEGNE (FR)**

(74) Mandataire: **Vial, Lionel et al
Cabinet Lionel Vial
6 rue de Vaugondran
91190 Gif-sur-Yvette (FR)**

(56) Documents cités:
**WO-A1-03/090776      WO-A1-2009/050580
WO-A2-2009/024429**

• SINDHU SANGEETA C ET AL: "Effect of feeding probiotic fermented indigenous food mixture on serum cholesterol levels in mice.", NUTRITION RESEARCH, vol. 23, no. 8, août 2003 (2003-08), pages 1071-1080, XP002594544, ISSN: 0271-5317
• PSOMAS E I ET AL: "Assimilation of Cholesterol by Yeast Strains Isolated from Infant Feces and Feta Cheese", JOURNAL OF DAIRY SCIENCE, AMERICAN DAIRY SCIENCE ASSOCIATION, US, vol. 86, no. 11, 1 novembre 2003 (2003-11-01), pages 3416-3422, XP026982828, ISSN: 0022-0302 [extrait le 2003-11-01]
• KRASOWSKA ANNA ET AL: "Assimilation of omega 3 and omega 6 fatty acids and removing of cholesterol from environment by Saccharomyces cerevisiae and Saccharomyces boulardii strains", JOURNAL OF BIOTECHNOLOGY, vol. 131, no. 2, Suppl. S, septembre 2007 (2007-09), pages S63-S64, XP002594545, & 13TH EUROPEAN CONGRESS ON BIOTECHNOLOGY (ECB 13); BARCELONA, SPAIN; SEPTEMBER 16 -19, 2007 ISSN: 0168-1656

**Description**

Domaine de l'invention

La présente invention concerne un médicament destiné à réduire la cholestérolémie et/ou la triglycéridémie chez un individu présentant des facteurs de risque cardiovasculaire ou à prévenir ou traiter l'hypercholestéromie et/ou l'hypertriglycéridémie chez un individu.

Arrière-plan technique

L'hypercholestérolémie est un facteur de risque majeur de maladies cardiovasculaires, comme l'athérosclérose, l'infarctus du myocarde, et les maladies cérébrovasculaires.

Le traitement de première intention pour l'hypercholestérolémie est un régime alimentaire adapté pauvre en cholestérol. Si la modification du régime alimentaire s'avère insuffisante pour traiter l'hypercholestérolémie, des traitements pharmacologiques sont alors envisagés, notamment à bases d'inhibiteurs de HMG-CoA réductase (statines) et de fibrates.

Divers autres moyens de prise en charge de l'hypercholestérolémie sont également disponibles, en particulier en complément du régime alimentaire adapté, comme les phytostérols, que l'on trouve incorporés dans de nombreuses préparations alimentaires, ou les probiotiques, notamment bactériens.

Du fait de leurs autres propriétés intéressantes en santé humaine et de leur quasi absence d'effets secondaires, il a ainsi été proposé d'utiliser de nombreux probiotiques différents dans le cadre du traitement de l'hypercholestérolémie. Cependant, cela repose le plus souvent sur des études *in vitro* d'extraction du cholestérol par les probiotiques à partir d'un milieu de culture. Or ces études ne sont pas directement indicatives d'un effet anti-hypercholestérolémique *in vivo*. Ainsi, sur la base d'étude *in vitro*, il pu être proposé que *Lactobacillus rhamnosus* puisse exercer un effet anti-hypercholestérolémique (Xanthopoulos et al. (1998) Microbiologie, Aliments, Nutrition 16:199-203), alors qu'aucun effet n'est observé *in vivo* (Hattaka et al. (2008) J. Am. Coll. Nutr. 27:441-447).

De fait, à l'heure actuelle, un véritable effet anti-hypercholestérolémique n'a pu être démontré de manière adéquate par des expériences *in vivo* que pour un faible nombre de probiotiques. On peut citer à ce titre, une association des probiotiques *Lactobacillus acidophilus* et *Streptococcus faecalis* (Fukushima & Nakano (1996) Br. J. Nutr. 76:857-867) ou une association de probiotiques composée de *Bacillus,* de *Lactobacillus,* de *Streptococcus,* de *Saccharomyces,* et de *Candida* (Fukushima & Nakano (1995) Br. J. Nutr. 73:701-710).

*Saccharomyces boulardii* (Ultra-Levure®) est une souche particulière de la levure *Saccharomyces cerevisiae.* Ce probiotique est principalement indiqué en complément de la réhydratation pour le traitement des diarrhées. Son utilité a été notamment établie chez l'enfant (Villarruel et al. (2007) Acta Paediatr 96:538-541 ; Szajewska et al. (2007) Aliment Pharmacol Ther 25:257-264) et pour les diarrhées liées à la prise d'antibiotiques (Surawicz et al. (1989) Gastroenterology 96:981-988; Kotowska et al. (2005) Aliment Pharmacol Ther 21:583-590) ou aux infections à *Clostridium difficile* (Surawicz et al. (2000) Clin Infect Dis 31:1012-1017).

Résumé de l'invention

La présente invention découle de la mise en évidence inattendue, par les inventeurs, que *Saccharomyces boulardii* permettait de réduire l'hypercholestérolémie et l'hépatomégalie induites chez le rat par un régime enrichi en cholestérol, ainsi que l'hypercholestérolémie et l'hypertriglycéridémie induites chez le hamster par un régime enrichi en cholestérol.

Ainsi, la présente invention concerne des cellules de levure *Saccharomyces boulardii* pour leur utilisation dans la réduction de la cholestérolémie et/ou de la triglycéridémie chez un individu présentant des facteurs de risque cardiovasculaire ou dans la prévention ou le traitement de l'hypercholestérolémie et/ou l'hypertriglycéridémie chez un individu.

La présente invention concerne également une composition pharmaceutique comprenant, à titre de substances actives :

- des cellules de levure *Saccharomyces boulardii,* et
- au moins un composé additionnel destiné au traitement de l'hypercholestérolémie et/ou de l'hypertriglycéridémie sélectionné dans le groupe constitué d'une statine, d'un fibrate et d'un phytostérol, éventuellement en association avec un véhicule pharmaceutiquement acceptable, en particulier pour son utilisation dans la réduction de la cholestérolémie et/ou de la triglycéridémie chez un individu, plus particulièrement pour traiter l'hypercholestérolémie et/ou l'hypertriglycéridémie.

La présente invention concerne également des produits contenant :

- des cellules de levure *Saccharomyces boulardii,* et
- au moins un composé additionnel destiné au traitement de l'hypercholestérolémie et/ou de l'hypertriglycéridémie,

comme produit de combinaison pour une utilisation simultanée, séparée ou étalée dans le temps pour la réduction de la cholestérolémie et/ou de la triglycéridémie chez un individu présentant des facteurs de risque cardiovasculaire, notamment pour traiter l'hypercholestérolémie et/ou l'hypertriglycéridémie.

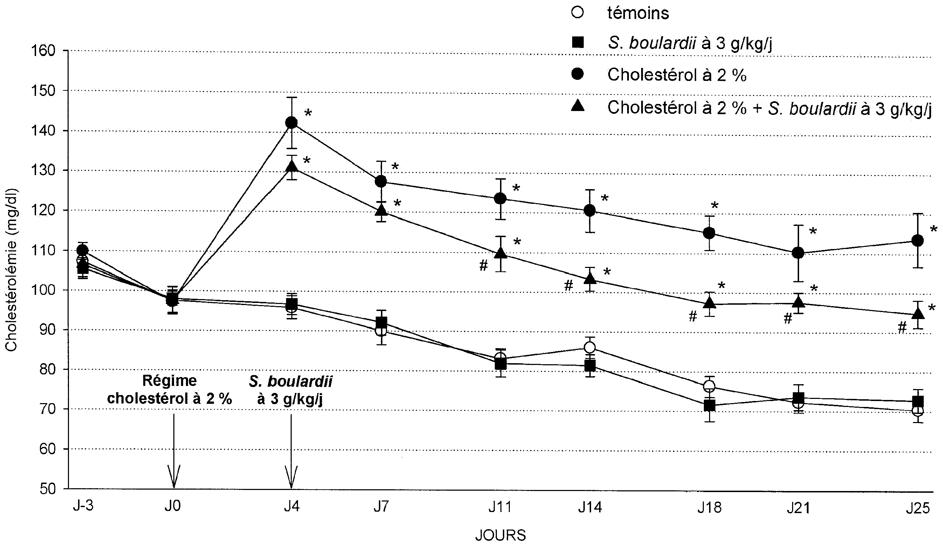

Description de la figure

La figure représente l'effet de *Saccharomyces boulardii* administré à 3 g/kg/j sur l'augmentation de la cholestérolémie de rats (axe des ordonnées, en mg/dl) induite par un régime alimentaire enrichi en cholestérol à 2%, en fonction du temps (axe des abscisses, en jours). Le régime enrichi en cholestérol et *S. boulardii* sont donnés à partir de J0 et J4 respectivement. * : $p < 0,05$, test statistique ANOVA avec mesures répétées des groupes traités comparés au groupe témoin et # : $p < 0,05$ pour le groupe *S. boulardii* + cholestérol comparé au groupe cholestérol seul. Le groupe de rats témoins est représenté par des cercles clairs, le groupe de rats recevant un régime alimentaire normal et *S. boulardii* est représenté par des carrés noirs, le groupe de rats recevant un régime alimentaire enrichi en cholestérol est représenté par des cercles noirs, et le groupe de rats recevant un régime alimentaire enrichi en cholestérol et *S. boulardii* est représenté par des triangles noirs.

Description détaillée de l'invention

La notion de « cholestérolémie » est bien connue de l'homme du métier. Elle représente un taux de cholestérol sanguin, et notamment le taux de cholestérol total sérique. La cholestérolémie est notamment exprimée en g/l ou en mmol/l.

Comme on l'entend ici, l'expression « réduire la cholestérolémie » signifie notamment, diminuer le taux sérique de cholestérol total.

Le terme « hypercholestérolémie » désigne une cholestérolémie pathologique. L'hypercholestérolémie désigne en particulier une cholestérolémie supérieure à 2 g/l, plus particulièrement supérieure à 2,4 g/l chez l'homme.

Les expressions « dans le traitement de l'hypercholestérolémie » et « pour traiter l'hypercholestérolémie » sont synonymes et signifient diminuer, en particulier pour la rendre normale, la cholestérolémie chez un individu présentant une hypercholestérolémie.

L'individu chez lequel on souhaite réduire la cholestérolémie peut présenter une hypercholestérolémie. L'individu peut également présenter une cholestérolémie qui n'est pas en elle-même pathologique, c'est-à-dire qui est de préférence inférieure ou égale à 2 g/l, mais qui est suffisamment élevée, pour rendre souhaitable une diminution de la cholestérolémie, par exemple si l'individu présente d'autres facteurs de risque cardiovasculaire ou en prévention d'une hypercholestérolémie. Ainsi, l'individu selon l'invention peut présenter une cholestérolémie supérieure à 1,8 g/l et inférieure ou égale à 2 g/l.

En particulier, l'individu selon l'invention peut présenter une hypercholestérolémie, notamment modérée, c'est-à-dire qu'il présente une cholestérolémie de préférence supérieure à 2 g/l et de préférence inférieure ou égale à 2,4 g/l, et est soumis à un régime alimentaire appauvri en cholestérol.

On désigne par « régime alimentaire appauvri en cholestérol » un régime dans lequel la quantité de cholestérol est diminuée par rapport à un régime alimentaire normal. L'homme du métier sait bien définir un régime appauvri en cholestérol. En particulier, dans un régime appauvri en cholestérol, la quantité de cholestérol est inférieure à la quantité de cholestérol qui se trouve habituellement dans le régime alimentaire de l'individu avant qu'il ne débute le régime appauvri en cholestérol.

La notion de « triglycéridémie » est également bien connue de l'homme du métier. Elle représente un taux de triglycérides sanguins, et notamment le taux de triglycérides total sérique. La triglycéridémie est notamment exprimée en g/l ou en mmol/l.

Comme on l'entend ici, l'expression « réduire la triglycéridémie » signifie notamment, diminuer le taux sérique de triglycérides totaux.

Le terme « hypertriglycéridémie » désigne une triglycéridémie pathologique. L'hypertriglycéridémie désigne en particulier une triglycéridémie supérieure à 1,5 g/l, plus particulièrement supérieure à 5 g/l.

Les expressions « dans le traitement de l'hypertriglycéridémie » et « pour traiter l'hypertriglycéridémie » sont synonymes et signifient diminuer, en particulier pour la rendre normale, la triglycéridémie chez un individu présentant une hypertriglycéridémie.

L'individu chez lequel on souhaite réduire la triglycéridémie peut présenter une hypertriglycéridémie. L'individu peut également présenter une triglycéridémie qui n'est pas en elle-même pathologique, c'est-à-dire qui est de préférence inférieure à 1,5 g/l, mais qui est suffisamment élevée, pour rendre souhaitable une diminution de la triglycéridémie, par exemple si l'individu présente d'autres facteurs de risque cardiovasculaire, ou en prévention d'une hypertriglycéridémie. Ainsi, l'individu selon l'invention peut présenter une triglycéridémie supérieure à 1,3 g/l et inférieure à 1,5 g/l pour un homme, ou supérieure à 1,1 g/l et inférieure à 1,5 g/l pour une femme.

L'individu selon l'invention est un animal, de préférence un mammifère, et plus préférablement un humain.

Comme on l'entend ici, l'expression « cellules de levure » regroupe des cellules de levure viables ou mortes, entières ou sous forme de débris. De préférence, au moins une partie des cellules de levure *Saccharomyces boulardii* selon l'invention sont viables.

La viabilité d'une cellule de levure est définie comme la capacité d'une cellule de levure à se multiplier. Le nombre de cellules viables dans un échantillon peut être estimé en déterminant le nombre d'Unités Formant Colonie (UFC) comprises dans l'échantillon.

A titre d'exemple, le nombre d'UFC de cellules de levure d'un échantillon liquide comprenant des levures peut être déterminé en étalant un volume déterminé de l'échantillon sur un milieu solide, par exemple gélosé, permettant la croissance des levures et en incubant le milieu solide pendant une durée, par exemple 48 h, et à une température, par exemple 30°C, permettant la croissance de colonies de levures. Le nombre de colonies rapporté au volume étalé sur le milieu solide permet de déterminer le nombre d'UFC compris dans l'échantillon. Un protocole détaillé de la détermination d'UFC conforme à l'invention est notamment décrit dans Toothaker & Elmer (1984) Antimicrobial Agents and Chemotherapy 26:552-556 au paragraphe « *Assay for S. boulardii* ». Par ailleurs, lorsque l'échantillon de levure se présente sous forme d'un solide, par exemple une poudre lyophilisée, on préfère déterminer le nombre d'UFC compris dans l'échantillon après avoir repris une masse déterminée de l'échantillon dans une solution aqueuse, notamment de l'eau distillée ou une solution à 0,9% de NaCl à pH 7.

Une « levure » selon l'invention est un champignon de préférence unicellulaire. Les cellules de levure selon l'invention sont de l'espèce *Saccharomyces boulardii*. *Saccharomyces boulardii* est bien connu de l'homme du métier et est notamment décrit dans Hennequin et al. (2001) J. Clin. Microbiol. 39:551-559.

De manière particulièrement préférée, les cellules de levure *Saccharomyces boulardii* selon l'invention sont obtenues à partir de médicaments de marque Ultra-Levure®, Bioflor®, Codex®, Econorm®, Enflor®, Enterol®, Florastor®, Floratil®, Florestor®, Inteflora®, Perenterol®, Perenteryl®, Precosa®, Reflor®, Ultra-Levura®, ou à partir des dépôts effectués à *l'American Type Culture Collection* (ATCC, USA) sous la référence 74012 ou à la Collection Nationale de Culture et de Microorganismes (CNCM, France) sous la référence I-745.

De préférence, également les cellules de levure *Saccharomyces boulardii* selon l'invention sont lyophilisées, telles que les cellules de levure *Saccharomyces boulardii* de marque Ultra-Levure®, Bioflor®, Codex®, Econorm®, Enflor®, Enterol®, Florastor®, Floratil®, Florestor®, Inteflora®, Perenterol®, Perenteryl®, Precosa®, Reflor®, ou Ultra-Levura®.

De manière avantageuse, la viabilité et la vitalité de cellules de levure obtenues à partir de lyophilisats sont supérieures à ce qui peut être obtenu à partir d'autre mode de conservation des cellules de levure.

Comme on l'entend ici, la « lyophilisation » est une méthode de conservation dans laquelle les cellules de levure sont congelées puis soumises à une sublimation de l'eau congelée qu'elles contiennent pour donner un lyophilisat sous forme d'une poudre de levure sèche contenant de préférence moins de 2% d'eau et plus préférablement moins de 1% d'eau. De préférence, les cellules de levure lyophilisées sont obtenues à partir de concentrés de cellules de levure. N'importe quel type de méthode de lyophilisation de cellules de levure connu de l'homme du métier peut être utilisé. Toutefois, les cellules de levure sont de préférence lyophilisées selon l'invention à l'aide de la méthode de lyophilisation suivante :

- cultiver les cellules de levure dans un milieu nutritif liquide jusqu'à ce que les cellules atteignent une phase stationnaire;
- concentrer les cellules de levure cultivées et congeler le concentré;
- lyophiliser le concentré.

De préférence les cellules de levure *Saccharomyces boulardii* selon l'invention sont administrées sous forme de gélules ou de sachets.

De préférence également, les cellules de levure *Saccharomyces boulardii* selon l'invention sont administrées à une dose de $0,5.10^8$ à $100.10^{10}$ UFC/kg/j ou à une dose de 0,00125 g/kg/j à 25 g/kg/j.

Par ailleurs, la composition pharmaceutique telle que définie ci-dessus, ou les produits tels que définis ci-dessus, comprennent de préférence une dose de 50 à 250 mg de cellules de levure *Saccharomyces boulardii*. En outre les cellules de levure *Saccharomyces boulardii* pour leur utilisation telle que définie ci-dessus sont de préférence administrée à une dose unitaire de 50 à 250 mg.

Comme l'homme du métier le comprendra, la quantité de cellules de levure à administrer par unité de masse (kg) se réfère à la masse de l'individu auquel sont destinées les cellules de levure. Par ailleurs, lorsque la quantité de cellules de levure à administrer est exprimée en unité de masse (g), les cellules de levure sont de préférence sous forme lyophilisée.

Comme on l'entend ici l'expression « un composé additionnel destiné au traitement de l'hypercholestérolémie » regroupe l'ensemble des composés utilisé pour réduire la cholestérolémie, notamment chez un individu souffrant d'hypercholestérolémie. Ces composés peuvent être de tout type et couvrent notamment les composés pharmacologiques, les probiotiques et les prébiotiques. On préfère, toutefois, que le composé additionnel destiné au traitement de l'hypercholestérolémie selon l'invention soit sélectionné dans le groupe constitué d'une statine, d'un fibrate, de niacine, et d'un phytostérol.

De façon similaire, l'expression « un composé additionnel destiné au traitement de l'hypertriglycéridémie » regroupe l'ensemble des composés utilisé pour réduire la triglycéridémie, notamment chez un individu souffrant d'hypertriglycéridémie. Ces composés peuvent être de tout type et couvrent notamment les composés pharmacologiques, les probiotiques et les prébiotiques. On préfère, toutefois, que le composé additionnel destiné au traitement de la triglycéridémie selon l'invention soit sélectionné dans le groupe constitué d'une statine, d'un fibrate, de niacine, et d'un phytostérol.

Toutefois, dans un mode de réalisation particulier de l'invention, les cellules de levure *S. boulardii* :

- pour leur utilisation, selon l'invention, dans la réduction de la cholestérolémie et/ou de la triglycéridémie chez un individu présentant des facteurs de risque cardiovasculaire, notamment pour traiter l'hypercholestérolémie et/ou l'hypertriglycéridémie, ou
- lorsqu'elles sont utilisées pour la réduction de la cholestérolémie et/ou de la triglycéridémie, notamment pour le traitement de l'hypercholestérolémie et/ou de l'hypertriglycéridémie, ne sont pas associées, ou administrées conjointement, à de la niacine ou à de l'acide linoléique, ou à un autre acide gras polyinsaturé, ou plus généralement ne sont pas associées, ou administrées conjointement, à un composé additionnel destiné au traitement de l'hypercholestérolémie et/ou de l'hypertriglycéridémie tel que défini ci-dessus. Autrement dit, dans un mode de réalisation particulier de l'invention, les cellules de levure *S. boulardii* pour leur utilisation selon l'invention ou lorsqu'elles sont utilisées selon l'invention, sont le seul principe actif destiné à réduire la cholestérolémie et/ou la triglycéridémie, notamment à traiter l'hypercholestérolémie et/ou l'hypertriglycéridémie.

Par ailleurs, dans un autre mode de réalisation particulier de l'invention, les cellules de levure *Saccharomyces boulardii* ne sont pas administrées avec un autre probiotique, et la composition pharmaceutique ne comprend pas de probiotique autre que *Saccharomyces boulardii.*

Comme on l'entend ici, le terme « probiotique » désigne tout microorganisme, de préférence viable, administré à un individu, notamment en vue d'améliorer son état de santé.

## EXEMPLES

### Exemple 1

#### 1. Matériels et méthodes

##### 1.1. Animaux

Des rats Sprague Dawley (Janvier), de poids compris entre 200 et 220 grammes au début de l'étude sont utilisés après acclimatation d'au moins 7 jours dans l'animalerie (t° = 22 $\pm$ 2°C ; hygrométrie : 50 $\pm$ 20 % ; nourriture SAFE « A04 » ; cycle nycthéméral 12h/12h).

##### 1.2. Protocole

###### 1.2.1. Régime alimentaire

4 groupes de 8 à 10 rats ont été respectivement soumis à :

- un régime alimentaire normal « A04 » (SAFE),
- un régime alimentaire normal avec *Saccharomyces boulardii* à 3 g/kg/j,
- un régime alimentaire incorporant 2% de cholestérol (MP Biomedicals, réf. 904691), et
- un régime alimentaire incorporant 2% de cholestérol (MP Biomedicals, réf. 904691) supplémenté avec *Saccharomyces boulardii* à 3 g/kg/j.

*1.2.2. Prélèvements sanguins*

Du sang est prélevé de la veine caudale à l'aide d'une aiguille Terumo 21 G (réf. NN2125R) et d'une seringue Terumo 2 ml (réf. 55-025) 3 jours avant le début de l'expérimentation pour déterminer la cholestérolémie basale. Un antihémorragique (Dycinone®) est utilisé pour arrêter l'éventuel écoulement de sang.

Le sang est versé dans un tube Sarstedt Multivette de 600µl contenant un activateur de coagulation (réf. 15.1670). Le tube est centrifugé à 10 000 g (14 000 rpm) pendant 5 min. à température ambiante dans une centrifugeuse *Centrifuge 5415C* Eppendorff. On récupère le sérum dans plusieurs micro-tubes Sarstedt de 0,5 ml (réf. 72.699), qui sont placés à -80°C avant analyse.

Différents prélèvement sont ensuite effectués le jour du début du régime enrichi en cholestérol (J0) puis 4, 7, 11, 14, 18, 21 et 25 jours après le début du régime enrichi en cholestérol.

*1.2.3. Dosage du cholestérol total par colorimétrie*

Une solution mère de cholestérol (Sigma, réf. C3045) à 3 g/l est réalisée en solubilisant 30 mg de cholestérol dans 10 ml d'une solution de déoxycholate de sodium à 150 g/l, à 40°C pendant 60 min. minimum. La solution de déoxycholate de sodium est obtenue par solubilisation de 15 g de déoxycholate de sodium dans 100 ml de NaCl à 0,9% puis en agitant à 40°C pendant 60 min. minimum.

Une gamme étalon est préparée par dilutions successives dans la solution de déoxycholate de sodium à partir de la solution mère pour obtenir des concentrations de 0,375 ; 0,75 ; 1,5 et 3 g/l de cholestérol.

10 µl de l'échantillon ou de l'étalon est mélangé à 1000 µl de réactif CHOL destiné à mesurer le cholestérol total (Ménarini, réf. 30990). 30 min. après, la mesure de l'absorbance est effectuée par rapport à un blanc réactif, à une température de 37°C, à une longueur d'onde de 510 nm, sur un spectrophotomètre SPEDCOR 2005 avec des cuves ayant un trajet optique de 1 cm (Spectrovettes Evergreen, Dutscher, réf. 064005).

*1.2.3. Détermination du poids du foie*

Au terme de l'étude (J25), les rats sont sacrifiés et leur foie est prélevé et pesé.

1.3. Produits

*Saccharomyces boulardii* lyophilisé (Ultralevure® lot 4325, Biocodex) est conservé entre 4 et 6°C, il est solubilisé dans de l'eau distillée.

Le cholestérol (Sigma, réf. C3045) est stocké à -20°C. Le déoxycholate de sodium est obtenu chez Fluka (réf. 30970).

Le réactif de dosage CHOL (Ménarini) est stocké entre 4 et 6°C.

1.4. Administration

*Saccharomyces boulardii* est administré par voie orale 4 jours après le début du régime alimentaire enrichi en cholestérol quand l'hypercholestérolémie est installée.

**2. Résultats**

Aucune différence significative n'est observée dans le poids des animaux en fonction du régime alimentaire auquel ils ont été soumis.

Les résultats sont résumés dans le **Tableau 1** suivant et dans la **Figure.**

**Tableau 1** : Effet de S. *boulardii* administré à 3 g/kg/j sur l'augmentation de la cholestérolémie (mg/dl) induite par un régime alimentaire enrichi en cholestérol à 2% :

| S. *boulardii* | Cholestérol 2% | n | J-3 | J0 | J4 | J7 | J11 | J14 | J18 | J21 | J25 |
|---|---|---|---|---|---|---|---|---|---|---|---|
| - | - | 10 | 107,3 ±2,9 | 97,6 ±3,4 | 95,9 ±3,0 | 90,0 ±3,4 | 83,1 ±2,5 | 86,0 ±2,7 | 76,3 ±2,7 | 72,3 ±2,7 | 70,4 ±2,9 |
| + | - | 9 | 105,6 ±2,5 | 98,0 ±1,7 | 96,7 ±2,6 | 92,1 ±3,1 | 81,9 ±3,4 | 81,5 ±2,8 | 71,6 ±4,2 | 73,7 ±3,3 | 72,8 ±3,0 |
| - | + | 8 | 109,9 ±2,2 | 97,3 ±2,8 | 142,3 ±6,4 * | 127,6 ±5,1 * | 123,5 ±5,1 * | 120,6 ±5,3 * | 115,2 ±4,3 * | 110,3 ±7,1 * | 113,5 ±6,8 * |
| + | + | 9 | 106,6 ±3,1 | 97,6 ±3,5 | 131,1 ±3,0 * | 120,1 ±2,5 * | 109,6 ±4,5 *# | 103,4 ±3,0 *# | 97,2 ±3,1 *# | 97,5 ±3,1 *# | 94,7 ±3,5 *# |
| Le régime enrichi en cholestérol et S. *boulardii* sont donnés à partir de J0 et J4 respectivement. * : $p < 0,05$, test statistique ANOVA avec mesures répétées des groupes traités comparés au groupe témoin et # : $p < 0,05$ pour le groupe S. *boulardii* + cholestérol comparé au groupe cholestérol seul. | | | | | | | | | | | |

Le régime enrichi en cholestérol entraine une augmentation significative de la cholestérolémie dès le premier dosage effectué à J4 avec un taux de cholestérol à 142,3 mg/dl, soit une augmentation de 48% de la cholestérolémie par rapport à celle du groupe témoins (95,9 mg/dl).

A partir de J11 et jusqu'à la fin de l'étude, la cholestérolémie du groupe *S. boulardii* + cholestérol est significativement diminuée par rapport au groupe cholestérol.

Par ailleurs, on observe une augmentation significative de 20% du poids moyen des foies du groupe cholestérol (16,2 ± 0,6 g) par rapport au groupe témoin (13,5 ± 0,4 g). *S. boulardii* est sans effet propre (13,0 ± 0,4 g). Par contre, pour le groupe *S. boulardii* + cholestérol, le poids du foie (14,9 ± 0,3 g) ne s'accroit, et de manière non-significative, que de 10%.

En conclusion, *S. boulardii* limite de manière significative l'hypercholestérolémie et l'hépatomégalie induites par un régime alimentaire enrichi en cholestérol chez le rat.

## Exemple 2

Dans cet exemple, les résultats obtenus chez le rat ont été confirmé chez le hamster, un autre modèle animal reconnu d'étude de l'hypercholestérolémie (Bhathena et al. (2009) J. Med. Food 12: 310-319 ; Chiu et al. (2006) Appl. Microbiol. Biotechnol. 71 : 238-245.).

## 1. Matériel et méthodes

### 1.1. Animaux

Des hamsters mâles syriens dorés (Janvier), de poids compris entre 70 et 80 grammes au début des études sont utilisés après acclimatation d'au moins 7 jours dans l'animalerie (t° = 22 ± 2°C ; nourriture SAFE "105" ; cycle nycthéméral 12h/12h).

### 1.2. Protocole

#### 1.2.1. Régime alimentaire enrichi en cholestérol

Différents lots de régime "105" enrichis en cholestérol à 0,1-0,25-0,5-1 % ont été préparé par SAFE. Les hamsters sont mis par 5 en cage au début de l'expérimentation. *Saccharomyces boulardii* Biocodex est administré par voie orale.

#### 1.2.2. Prélèvements sanguin et hépatique

Les hamsters sont mis à jeun de nourriture la veille des prélèvements.

A la fin de l'expérimentation, on prélève du sang par ponction cardiaque, sous anesthésie à l'isoflurane. Ensuite le foie est prélevé, puis il est congelé à -80°C pour des analyses ultérieures.

Le sang est versé dans un tube Sarstedt Multivette de 600 µl contenant un activateur de coagulation (réf 15.1670). Le tube est centrifugé à 10 000 g (14 000 rpm) pendant 5 minutes à température ambiante dans la centrifugeuse Centrifuge 5415C Eppendorff. On récupère le sérum dans plusieurs micro-tubes Sarstedt de 0.5 ml (réf 72.699) qui sont placés dans un congélateur à -80°C avant analyses.

#### 1.2.3. Dosage du cholestérol sérique par colorimétrie

Une solution mère de cholestérol (Sigma, réf. C3045) à 3 g/l est réalisée en solubilisant 30 mg de cholestérol dans 10 ml d'une solution de déoxycholate de sodium à 150 g/l, à 40°C pendant 60 min. minimum. La solution de déoxycholate de sodium est obtenue par solubilisation de 15 g de déoxycholate de sodium dans 100 ml de NaCl à 0,9% puis en agitant à 40°C pendant 60 min. minimum.

Une gamme étalon est préparée par dilutions successives dans la solution de déoxycholate de sodium à partir de la solution mère pour obtenir des concentrations de 0,375 ; 0,75 ; 1,5 et 3 g/l de cholestérol.

10 µl de l'échantillon ou de l'étalon est mélangé à 1000 µl de réactif destiné à mesurer le cholestérol total (Ménarini, réf. 30990). 30 min. après, la mesure de l'absorbance est effectuée par rapport à un blanc réactif, à une température de 37°C, à une longueur d'onde de 510 nm, sur un spectrophotomètre SPEDCOR 2005 avec des cuves ayant un trajet optique de 1 cm (Spectrovettes Evergreen, Dutscher, réf. 064005).

Des contrôles sont effectués avec le kit multicalibrateur (Ménarini réf 37484), le kit contrôle bas (réf 37492) et le kit contrôle haut (réf 37493).

*1.2.4. Dosage du cholestérol hépatique par colorimétrie*

Le protocole est adapté de Loison et al. (2002) Reprod. Nutr. Dev. 42:101-14. 1 g de foie est décongelé puis broyé et homogénéisé dans 5 ml d'isopropanol (tube Sarstedt 50 ml), à l'aide d'un broyeur Ultra-Turax. Le broyat est incubé à 60°C pendant 1 h sous agitation (250 rpm, NBS incubateur shaker C24) puis centrifugé à 3000 g pendant 5 min à 4°C (Beckmann Allegra 21 R rotor C0650). Le surnageant est récupéré (surnageant 1) et le culot est repris dans 5 ml d'isopropanol, puis centrifugé (3000 g, 5 min, à 4°C). Le surnageant obtenu (surnageant 2) est mélangé avec le surnageant 1, puis centrifugé à nouveau. Des aliquotes de 1,7 ml du surnageant ainsi obtenu sont stockés au congélateur à -80°C avant analyse si besoin.

10 $\mu$l du mélange des 2 surnageants de l'extrait de foie sont analysés comme indiqué ci-dessus pour le cholestérol sérique total.

*1.2.5. Dosage du HDL-cholestérol sérique*

Le test consiste en 2 étapes de réaction distinctes à l'aide du kit Ménarini HDL-cholestérol réf. 37459. Il y a tout d'abord une élimination des chylomicrons, du VLDL-cholestérol et du LDL-cholestérol par action d'une cholestérol estérase, une cholestérol oxydase et d'une catalase (réactif R1), puis mesure spécifique du HDL-cholestérol après libération du HDL-cholestérol par les détergents contenus dans le réactif R2. L'intensité du colorant "quinone imine" est directement proportionnelle à la concentration en cholestérol mesuré à 600 nm.

Pour la réalisation d'une courbe étalon, des dilutions successives d'une solution mère contenant 3,66 mM (soit 141,2 mg/dl) de HDL-cholestérol (kit de alibration HDL Ménarini, réf. 37485) sont réalisées dans de l'eau distillée afin d'obtenir des concentrations de 4,41 - 8,82 - 17,6 - 35,3 - 70.6 - 141.2 mg/dl.

Pour les mesures, on mélange 575 $\mu$l de réactif R1 avec 10 $\mu$l d'étalon ou d'échantillon (préalablement dilué de moitié) dans une cuvette, puis on laisse incuber 5 minutes avec agitation. On ajoute alors 175 $\mu$l de réactif R2 et l'on agite pendant 5 minutes avant de mesurer l'absorbance contre un blanc réactif. La température de travail est de 37°C et l'absorbance est mesurée à une longueur d'onde de 600 nm et avec des cuves spectrophotomètre d'un trajet optique de 1 cm (Spectrovettes Evergreen semi-micro haute précision à usage unique, réf 064005).

*1.2.6. Dosage des triglycérides sériques*

La détermination des triglycérides se fait après séparation enzymatique du glycérol avec la lipoproteine-lipase. Le produit coloré est une quinone-imine qui est mesurée à 510 nm, et qui est directement proportionnelle à la concentration de triglycérides dans l'échantillon.

Pour la réalisation d'une courbe étalon, des dilutions successives d'une solution mère de glycérol à 2.5 mg/ml sont réalisées dans de l'eau distillée afin d'obtenir des concentrations de 0, 31.25, 62.5, 125 et 250 mg/dl.

Les mesures sont réalisées à l'aide du kit triglycéride de Ménarini (réf. 37481) en mélangeant 10 $\mu$l d'échantillon ou d'étalon avec 1000 $\mu$l de réactif Ménarini. L'absorbance est déterminée après 30 min. par rapport à un blanc réactif. La température de travail est de 20-25°C et l'absorbance est mesurée à une longueur d'onde de 510 nm et avec des cuves spectrophotomètre d'un trajet optique de 1 cm (Spectrovettes Evergreen semi-micro haute précision à usage unique, réf 064005).

### 1.3. Produits

*Saccharomyces boulardii* lyophilisé (Ultralevure® lot 4325, Biocodex) est conservé entre 4 et 6°C, il est solubilisé dans de l'eau distillée.

Le cholestérol (Sigma, réf. C3045) est stocké à -20°C. Le déoxycholate de sodium est obtenu chez Fluka (réf. 30970).

L'isopropanol est obtenu chez VWR (réf 20880.290).

Glycérol (5 ml, à 2.5 mg/ml) est obtenu chez Sigma (réf G7793) et est stocké à 2-8°C.

Les kits de dosage Ménarini (Réactif de mesure du cholestérol total, réf 30990 ; kit cholestérol, réf 90132 ; kit triglycérides, réf 37481 ; kit HDL-cholestérol, réf 37459 ; kit multicalibrateur, réf 37484; kit contrôle bas, réf 37492; kit contrôle haut, réf 34493 ; réactif LDL/HDL, réf 37485) sont gardés entre 4 et 6°C.

### 1.4. Analyse statistique

Les résultats sont exprimés en moyenne $\pm$ SEM. Le test statistique utilisé est une analyse de variance à un facteur ou deux facteurs, sauf pour le paramètre des poids corporels. Quand le résultat ne dépend pas du hasard (à 5 %), on détermine les groupes traités qui diffèrent du groupe témoin à l'aide du test de Student Newman-Keuls ou de Bonferoni et du logiciel Sigma Stat (version 3.1). Pour les poids corporels, le test statistique utilisé est une analyse de variance avec mesures répétées.

**2. Résultats**

2.1. Etude N°1

Dans cette première étude (n = 5 par groupe), un régime enrichi en cholestérol à 0,1-0,5-1 % a été administré pendant 14 ou 28 jours pour mesurer l'effet de chacun de ces 3 régimes sur la cholestérolémie chez le hamster.

On ne constate aucune modification significative induite par les 3 régimes enrichis par rapport au groupe témoins en ce qui concerne l'évolution des poids corporels

La cholestérolémie basale se situe à $144 \pm 10$ mg/dl au début de l'étude (J0). Dans les 2 groupes témoins, la cholestérolémie diminue légèrement à $137 \pm 6$ mg/dl à J14 et à $125 \pm 11$ mg/dl à J28, mais non significativement.

Le régime enrichi à 0,1 % de cholestérol entraîne une augmentation de 36% de la cholestérolémie mesurée à J14 à $186 \pm 4$ mg/dl, et un accroissement significatif de 65% à J28 ($206 \pm 9$ mg/dl). Aux concentrations supérieures de 0,5 et 1% de cholestérol dans le régime, une augmentation encore plus forte de la cholestérolémie est constatée.

De plus, le régime enrichi à 0.1 % de cholestérol entraîne une augmentation significative du cholestérol hépatique. Une augmentation encore plus forte du cholestérol hépatique est observée pour les régimes enrichis par des concentrations de 0,5 et 1% de cholestérol.

Cette première étude montre qu'un régime alimentaire enrichi à partir de 0,1 % de cholestérol permet d'obtenir une hypercholestérolémie significative chez le hamster.

2.2. Etude n°2

Dans cette étude (n = 10 par groupe), un régime enrichi en cholestérol à 0,1% (SAFE) a été administré pendant 14 jours pour mesurer l'effet de *Saccharomyces boulardii* Biocodex à 2x3 g/kg/j sur l'hypercholestérolémie.

On ne constate aucune modification significative du poids corporel induite par le régime enrichi et/ou par *Saccharomyces boulardii* Biocodex administré à 2x3 g/kg/j par rapport au groupe témoins.

La cholestérolémie du groupe témoin est de $138 \pm 3$ mg/dl **(Tableau 2).** Le régime enrichi à 0,1% de cholestérol entraîne une augmentation significative de 55% de la cholestérolémie mesurée à J14 ($214 \pm 11$ mg/dl). *Saccharomyces boulardii* Biocodex administré à 2x3 g/kg/j ne modifie pas la cholestérolémie basale ($134 \pm 3$ mg/dl), mais la levure limite l'augmentation de la cholestérolémie du groupe recevant le régime enrichi à seulement 33% ($184 \pm 3$ mg/dl). *Saccharomyces boulardii* Biocodex diminue donc significativement de 14% l'augmentation de la cholestérolémie induite par le régime enrichi en cholestérol à 0,1%.

De même, le régime enrichi augmente significativement le cholestérol hépatique de 387% : $3,22 \pm 0.09$ mg de cholestérol par gramme de foie dans le groupe témoin et $15.67 \pm 0.78$ mg/g dans le groupe recevant le régime enrichi. *Saccharomyces boulardii* Biocodex administré à 2x3 g/kg/j ne modifie pas le cholestérol hépatique basal ($2,76 \pm 0.13$ mg/g), mais la levure limite l'augmentation du cholestérol hépatique du groupe recevant le régime enrichi à seulement 261% ($11,62 \pm 0,79$ mg/g). *Saccharomyces boulardii* Biocodex diminue donc significativement de 26% l'augmentation du cholestérol hépatique induite par le régime enrichi en cholestérol à 0,1%.

2.3. Etude N°3

Dans cette étude (n = 10), le même protocole que pour l'étude précédente a été reproduit, en utilisant une dose inférieure de *Saccharomyces boulardii* Biocodex à 2 x 1 g/kg/j.

On ne constate aucune modification significative du poids corporel induite par le régime enrichi et/ou par *Saccharomyces boulardii* Biocodex administré à 2 x 1 g/kg/j par rapport au groupe Témoins.

*Dosage du cholestérol*

La cholestérolémie du groupe témoin est de $132 \pm 5$ mg/dl **(Tableau 2).** Le régime enrichi à 0,1% de cholestérol entraîne une augmentation significative de 52% de la cholestérolémie mesurée à J14 ($200 \pm 10$ mg/dl). *Saccharomyces boulardii* Biocodex administré à 2 x 1 g/kg/j ne modifie pas la cholestérolémie basale ($129 \pm 6$ mg/dl), mais la levure limite l'augmentation de la cholestérolémie du groupe recevant le régime enrichi à seulement 34% ($176 \pm 4$ mg/dl). *Saccharomyces boulardii* Biocodex diminue significativement de 12% l'augmentation de la cholestérolémie induite par le régime enrichi en cholestérol à 0,1 %.

*Dosage du HDL-cholestérol*

La concentration sérique en HDL-cholestérol du groupe témoin est de 76,3±1,8 mg/dl **(Tableau 2)**. Le régime enrichi à 0,1% de cholestérol entraîne une augmentation significative de 34% du HDL-cholestérol sérique mesuré à J14 (102.2±6.1 mg/dl). *Saccharomyces boulardii* Biocodex administré à 2 x 1 g/kg/j ne modifie pas la concentration basale en HDL-cholestérol (72.7±2.4 mg/dl), mais la levure limite l'augmentation du HDL-cholestérol du groupe recevant le régime enrichi à seulement 18% (90.4±3.1 mg/dl). *Saccharomyces boulardii* Biocodex diminue donc significativement l'augmentation du HDL-cholestérol induite par le régime enrichi en cholestérol à 0,1%.

*Calcul du VLDL+LDL-cholestérol*

Le cholestérol sous forme non-HDL, c'est-à-dire le cholestérol sous forme VLDL (*very low density lipoprotein*) et LDL, a été calculé selon la formule :

$$\text{Non-HDL-Cholestérol} = \text{Cholestérol Total} - \text{HDL-Cholestérol}$$

La concentration sérique en VLDL+LDL-cholestérol du groupe témoin est de 55,3±3,5 mg/dl. Le régime enrichi à 0,1% de cholestérol entraîne une augmentation significative de 75% du VLDL+LDL-cholestérol sérique mesuré à J14 (96,6±5,5 mg/dl). *Saccharomyces boulardii* Biocodex administré à 2 x 1 g/kg/j ne modifie pas la concentration basale en VLDL+LDL-cholestérol (56,5±4,5 mg/dl), mais la levure limite l'augmentation du VLDL+LDL-cholestérol du groupe recevant le régime enrichi à seulement 55% (85,7±3,0 mg/dl).

*Dosage des triglycérides*

La concentration sérique en triglycérides du groupe témoin est de 214±33 mg/dl **(Tableau 2)**. Le régime enrichi à 0,1% de cholestérol entraîne une augmentation significative de 82% des triglycérides sériques mesurés à J14 (389±75 mg/dl). *Saccharomyces boulardii* Biocodex administré à 2 x 1 g/kg/j ne modifie pas la concentration basale en triglycérides (222±28 mg/dl), mais la levure bloque complètement l'augmentation des triglycérides sériques du groupe recevant le régime enrichi (251±32 mg/dl).

2.4. Etude N°4

Dans cette étude (n = 10), *Saccharomyces boulardii* Biocodex a été administré à 2 x 3 g/kg/j à partir du 14ème jour après le début du régime enrichi en cholestérol à 0,1%, et pendant 14 jours, pour étudier l'effet de la levure sur une hypercholestérolémie déjà établie.
On ne constate aucune modification significative du poids corporel induite par le régime enrichi et/ou par *Saccharomyces boulardii* Biocodex administré à 2 x 3 g/kg/j par rapport au groupe témoins.

*Dosage du cholestérol*

La cholestérolémie du groupe témoin est de 111±3 mg/dl **(Tableau 2)**. Le régime enrichi à 0,1% de cholestérol entraîne une augmentation significative de 108% de la cholestérolémie mesurée à J28 (231±10 mg/dl). *Saccharomyces boulardii* Biocodex administré à 2 x 3 g/kg/j à partir du 14ème jour ne modifie pas la cholestérolémie basale (116±4 mg/dl), mais la levure limite l'augmentation de la cholestérolémie du groupe recevant le régime enrichi à seulement 82% (203±5 mg/dl). Lorsque l'hypercholestérolémie est établie, *Saccharomyces boulardii* Biocodex est donc capable de diminuer significativement de 12% l'augmentation de la cholestérolémie induite par le régime enrichi en cholestérol à 0.1% au bout de 14 jours.

*Dosage du HDL-cholestérol*

La concentration sérique en HDL-cholestérol du groupe témoin est de 73,3±2,1 mg/dl **(Tableau 2)**. Le régime enrichi à 0,1% de cholestérol entraîne une augmentation significative de 61% du HDL-cholestérol sérique mesuré à J28 (118.0.2±3.2 mg/dl). *Saccharomyces boulardii* Biocodex administré à 2 x 3 g/kg/j, à partir du 14ème jour ne modifie pas la concentration basale en HDL-cholestérol (77.0±2.5 mg/dl), mais la levure limite l'augmentation du HDL-cholestérol du groupe recevant le régime enrichi à seulement 43% (105.0±2.6 mg/dl). Lorsque l'hypercholestérolémie est établie, *Saccharomyces boulardii* est donc capable de diminuer significativement de 11% l'augmentation du HDL-cholestérol induite par le régime enrichi en cholestérol à 0,1 % au bout de 14 jours.

*Calcul du VLDL+LDL-cholestérol*

La concentration sérique en VLDL+LDL-cholestérol du groupe témoin est de 38,0±3,0 mg/dl. Le régime enrichi à 0.1% de cholestérol entraîne une augmentation significative de 189% du VLDL+LDL-cholestérol sérique mesuré à J28 (110,0±7,7 mg/dl). *Saccharomyces boulardii* Biocodex administré à 2 x 3 g/kg/j ne modifie pas la concentration basale en VLDL+LDL-cholestérol (38.9±2.9 mg/dl), mais la levure limite l'augmentation du VLDL+LDL-cholestérol du groupe recevant le régime enrichi à seulement 158% (98.0±3.8 mg/dl).

*Dosage des triglycérides*

La concentration sérique en triglycérides du groupe témoin est de 151±31 mg/dl **(Tableau 2)**. Le régime enrichi à 0,1% de cholestérol entraîne une augmentation significative de 130% des triglycérides sériques mesurés à J28 (348±66 mg/dl). *Saccharomyces boulardii* Biocodex administré à 2 x 3 g/kg/j, à partir du 14ème jour quand l'hypercholestérolémie est présente, ne modifie pas la concentration basale en triglycérides (132±20 mg/dl), mais la levure bloque complètement l'augmentation des triglycérides sériques du groupe recevant le régime enrichi (212±25 mg/dl).

En conclusion, l'administration de *Saccharomyces boulardii* Biocodex au moment de l'installation d'une hypercholestérolémie, ou lorsque une hypercholestérolémie est installée, entraîne une réduction significative de l'hypercholestérolémie induite par un régime alimentaire enrichi en cholestérol chez le hamster. *Saccharomyces boulardii* Biocodex permet également de s'opposer de manière significative à l'augmentation de la concentration en triglycérides sériques induite par le régime alimentaire enrichi en cholestérol chez le hamster.

**Tableau 2 :** Récapitulatif des effets de *Saccharomyces boulardii* Biocodex sur les paramètres de la cholestérolémie et de la triglycéridémie modifiés par un régime enrichi à 0,1 % de cholestérol chez le hamster.

| Régime enrichi en cholestérol | *Saccharomyces boulardii* | Cholestérolémie (mg/dl) | % var | HDL-cholestérol (mg/dl) | % var | Triglycérides (mg/dl) | % var |
|---|---|---|---|---|---|---|---|
| **De J0 à J14** | **De J0 à J14** | | | | | | |
| - | - | 138 ± 3 | | | | | |
| 0.1 % | - | 214 ± 11* | + 55 | | | | |
| 0 | 2 x 3 g/kg/j | 134 ± 3 | + 3 | | | | |
| 0.1 % | 2 x 3 g/kg/j | 184 ± 3* @ | + 33 | | | | |
| **De J0 à J14** | **De J0 à J14** | | | | | | |
| - | - | 132 ± 5 | | 76.3 ± 1.8 | | 214 ± 33 | |
| 0.1 % | - | 200 ± 10 * | + 52 | 102.2 ± 6.1* | + 34 | 389 ± 75* | + 82 |
| 0 | 2 x 1 g/kg/j | 129. ± 6 | - 2 | 72.7 ± 2.4 | - 5 | 222 ± 28 | + 4 |
| 0.1 % | 2 x 1 g/kg/j | 176 ± 4 * @ | + 34 | 90.4 ± 3.1* @ | + 18 | 251 ± 32 @ | + 17 |
| **De J0 à J28** | **De J14 à J28** | | | | | | |
| - | - | 111 ± 3 | | 73.3 ± 2.1 | | 151 ± 31 | |
| 0.1 % | - | 231 ± 10* | + 108 | 118.0 ± 3.2* | + 61 | 348 ± 66* | + 130 |
| 0 | 2 x 3 g/kg/j | 116 ± 4 | + 4 | 77.0 ± 2.5 | + 5 | 132 ± 20 | - 13 |
| 0.1 % | 2 x 3 g/kg/j | 203 ± 5* @ | + 82 | 105.0 ± 2.6* @ | + 43 | 212 ± 25 @ | + 40 |

(* : test statistique ANOVA à 2 facteurs des groupes traités par rapport aux groupes contrôles respectifs; @ : test statistique ANOVA à 2 facteurs des groupes recevant *S. boulardii* Biocodex et le régime enrichi par rapport aux groupes respectifs recevant seulement le régime enrichi). (% var : pourcentage de variation par rapport au groupe témoin sans traitement)

**Revendications**

1. Cellules de levure *Saccharomyces boulardii* pour leur utilisation dans la réduction de la cholestérolémie et/ou de la triglycéridémie chez un individu présentant des facteurs de risque cardiovasculaire ou dans la prévention ou le traitement de l'hypercholestérolémie et/ou de l'hypertriglycéridémie chez un individu.

**2.** Cellules de levure *Saccharomyces boulardii* pour leur utilisation selon la revendication 1, les cellules de levure étant lyophilisées.

**3.** Cellules de levure *Saccharomyces boulardii* pour leur utilisation selon la revendication 1 ou 2, les cellules de levure *Saccharomyces boulardii* étant administrées sous forme de gélules ou de sachets.

**4.** Cellules de levure *Saccharomyces boulardii* pour leur utilisation selon l'une des revendications 1 à 3, les cellules de levure *Saccharomyces boulardii* étant administrées à une dose de 0,00125 à 25 g/kg/j.

**5.** Composition pharmaceutique comprenant à titre de substances actives :

- des cellules de levure *Saccharomyces boulardii,* et
- au moins un composé additionnel destiné au traitement de l'hypercholestérolémie et/ou de l'hypertriglycéridémie sélectionné dans le groupe constitué d'une statine, d'un fibrate et d'un phytostérol,

éventuellement en association avec un véhicule pharmaceutiquement acceptable.

**6.** Composition pharmaceutique selon la revendication 5, comprenant une dose de 50 à 250 mg de cellule de levure de l'espèce *Saccharomyces boulardii* sous forme lyophilisée.

**7.** Composition pharmaceutique selon la revendication 5 ou 6, pour réduire la cholestérolémie et/ou la triglycéridémie chez un individu.

**8.** Composition pharmaceutique selon l'une des revendications 5 à 7, pour son utilisation dans le traitement de l'hypercholestérolémie et/ou de l'hypertriglycéridémie.

**9.** Produits contenant :

- des cellules de levure *Saccharomyces boulardii,* et
- au moins un composé additionnel destiné au traitement de l'hypercholestérolémie et/ou de l'hypertriglycéridémie,

comme produit de combinaison pour une utilisation simultanée, séparée ou étalée dans le temps pour la réduction de la cholestérolémie et/ou de la triglycéridémie chez un individu présentant des facteurs de risque cardiovasculaire ou pour le traitement de l'hypercholestérolémie et/ou de l'hypertriglycéridémie chez un individu.

**10.** Produits pour une utilisation selon la revendication 9, dans lesquels le composé destiné au traitement de l'hypercholestérolémie et/ou de l'hypertriglycéridémie est sélectionné dans le groupe constitué d'une statine, d'un fibrate, de niacine, et d'un phytostérol.

**11.** Produits pour une utilisation selon la revendication 9 ou 10, les cellules de levure *Saccharomyces boulardii* étant lyophilisées.

**12.** Produits pour une utilisation selon l'une des revendications 9 à 11, les cellules de levure *Saccharomyces boulardii* étant administrées à une dose de 0,00125 à 25 g/kg/j.

**Patentansprüche**

**1.** Hefezellen *Saccharomyces boulardii* zur Anwendung zur Verringerung von Cholesterinämie und/oder von Triglyzeridämie bei einem Patienten, der Risikofaktoren für Herz-Kreislauferkrankungen aufweist, oder in der Vorbeugung oder Behandlung von Hypercholesterinämie und/oder von Hypertriglyzeridämie bei einem Patienten.

**2.** Hefezellen *Saccharomyces boulardii* zur Anwendung nach Patentanspruch 1, wobei die Hefezellen lyophilisiert sind.

**3.** Hefezellen *Saccharomyces boulardii* zur Anwendung nach Patentanspruch 1 oder 2, wobei die Hefezellen *Saccharomyces boulardii* in Form von Gelkapseln oder Tütchen verabreicht werden.

4. Hefezellen *Saccharomyces boulardii* zur Anwendung nach irgendeinem der Patentansprüche 1 bis 3, wobei die Hefezellen *Saccharomyces boulardii* in einer Dosis von 0,00125 bis 25 g/kg/j verabreicht werden.

5. Pharmazeutische Zubereitung, die als Wirkstoffe enthält

   - Hefezellen *Saccharomyces boulardii,* und
   - mindestens eine zusätzliche Verbindung zur Behandlung von Hypercholesterinämie und/oder von Hypertriglyzeridämie, ausgewählt aus der Gruppe, bestehend aus einem Statin, einem Fibrat und einem Phytosterol,

   gegebenenfalls in Verbindung mit einem pharmazeutisch geeigneten Vehikel.

6. Pharmazeutische Zubereitung nach Patentanspruch 5, eine Dosis von 50 bis 250 mg Hefezellen der Art *Saccharomyces boulardii* in lyophilisierter Form umfassend.

7. Pharmazeutische Zubereitung nach Patentanspruch 5 oder 6 zur Verringerung der Cholesterinämie und/oder der Triglyzeridämie bei einem Patienten.

8. Pharmazeutische Zubereitung nach irgendeinem der Patentansprüche 5 bis 7 zur Anwendung in der Behandlung von Hypercholesterinämie und/oder von Hypertriglyzeridämie.

9. Produkte, enthaltend:

   - Hefezellen *Saccharomyces boulardii,* und
   - mindestens eine zusätzliche Verbindung zur Behandlung von Hypercholesterinämie und/oder von Hypertriglyzeridämie,

   als Kombinationspräparate zur gleichzeitigen, gesonderten oder über einen Zeitraum verteilten Anwendung zur Verringerung der Cholesterinämie und/oder von Triglyzeridämie bei einem Patienten, der Risikofaktoren für Herz-Kreislauferkrankungen aufweist, oder in der Behandlung von Hypercholesterinämie und/oder von Hypertriglyzeridämie bei einem Patienten.

10. Produkte zur Anwendung nach Patentanspruch 9, in denen die Verbindung zur Behandlung von Hypercholesterinämie und/oder von Hypertriglyzeridämie aus der Gruppe, bestehend aus einem Statin, einem Fibrat, einem Niacin und einem Phytosterol, ausgewählt wurde.

11. Produkte zur Anwendung nach Patentanspruch 9 oder 10, in denen die Hefezellen *Saccharomyces boulardii* lyophilisiert sind.

12. Produkte zur Anwendung nach irgendeinem der Patentansprüche 9 bis 11, wobei die Hefezellen *Saccharomyces boulardii* in einer Dosis von 0,00125 bis 25 g/kg/j verabreicht werden.

**Claims**

1. *Saccharomyces boulardii* yeast cells for use in the reduction of blood cholesterol level and/or blood triglyceride level in an individual with cardiovascular risk factors or for the prevention or treatment of hypercholesterolemia and/or hypertriglyceridemia in an individual.

2. *Saccharomyces boulardii* yeast cells for use according to claim 1, the yeast cells being lyophilized.

3. *Saccharomyces boulardii* yeast cells for use according to claim 1 or 2, the *Saccharomyces boulardii* yeast cells being administered in the form of capsules or sachets.

4. *Saccharomyces boulardii* yeast cells for use according to any of claims 1 to 3, the *Saccharomyces boulardii* yeast cells being administered at a dose of from 0.00125 to 25 g/kg/d.

5. A pharmaceutical composition comprising as active ingredients:

- *Saccharomyces boulardii* yeast cells, and
- at least one additional compound intended for the treatment of hypercholesterolemia and/or hypertriglyceridemia selected from the group consisting of a statin, a fibrate and a phytosterol,

optionally in association with a pharmaceutically acceptable vehicle.

6. The pharmaceutical composition according to claim 5, comprising a dose of from 50 to 250 mg of yeast cells of the *Saccharomyces boulardii* species in lyophilized form.

7. The pharmaceutical composition according to claim 5 or 6, for reducing blood cholesterol level and/or blood triglyceride level in an individual.

8. The pharmaceutical composition according to any of claims 5 to 7, for use in the treatment of hypercholesterolemia and/or hypertriglyceridemia.

9. Products containing:

- *Saccharomyces boulardii* yeast cells, and
- at least one additional compound intended for the treatment of hypercholesterolemia and/or hypertriglyceridemia,

as a combined preparation for simultaneous, separate or sequential use for reducing blood cholesterol level and/or blood triglyceride level in an individual with cardiovascular risk factors or for treating hypercholesterolemia and/or hypertriglyceridemia in an individual.

10. Products for use according to claim 9, wherein the compound intended for the treatment of hypercholesterolemia and/or hypertriglyceridemia is selected from the group consisting of a statin, a fibrate, niacin, and a phytosterol.

11. Products for use according to claim 9 or 10, the *Saccharomyces boulardii* yeast cells being lyophilized.

12. Products for use according to any of claims 9 to 11, the *Saccharomyces boulardii* yeast cells being administered at a dose of from 0.00125 to 25 g/kg/d.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **XANTHOPOULOS et al.** *Microbiologie, Aliments, Nutrition,* 1998, vol. 16, 199-203
- **HATTAKA et al.** *J. Am. Coll. Nutr.,* 2008, vol. 27, 441-447
- **FUKUSHIMA ; NAKANO.** *Br. J. Nutr.,* 1996, vol. 76, 857-867
- **FUKUSHIMA ; NAKANO.** *Br. J. Nutr.,* 1995, vol. 73, 701-710
- **VILLARRUEL et al.** *Acta Paediatr,* 2007, vol. 96, 538-541
- **SZAJEWSKA et al.** *Aliment Pharmacol Ther,* 2007, vol. 25, 257-264
- **SURAWICZ et al.** *Gastroenterology,* 1989, vol. 96, 981-988
- **KOTOWSKA et al.** *Aliment Pharmacol Ther,* 2005, vol. 21, 583-590
- **SURAWICZ et al.** *Clin Infect Dis,* 2000, vol. 31, 1012-1017
- **TOOTHAKER ; ELMER.** *Antimicrobial Agents and Chemotherapy,* 1984, vol. 26, 552-556
- **HENNEQUIN et al.** *J. Clin. Microbiol.,* 2001, vol. 39, 551-559
- **BHATHENA et al.** *J. Med. Food,* 2009, vol. 12, 310-319
- **CHIU et al.** *Appl. Microbiol. Biotechnol.,* 2006, vol. 71, 238-245
- **LOISON et al.** *Reprod. Nutr. Dev.,* 2002, vol. 42, 101-14